# EUROPEAN PATENT SPECIFICATION

(11) **EP 1 244 638 B1**
(45) Date of publication and mention of the grant of the patent: **12.05.2004**
(21) Application number: 00986451.3
(22) Date of filing: 18.12.2000
(51) Int. Cl.: C07D 241/04, A61K 31/495, A61P 25/00

(54) **PHENANTHRYL PIPERAZINYL DICARBOXYLIC ACIDS AS SELECTIVE NMDA RECEPTOR MODULATING AGENTS**
PHENANTHRYL-PIPERAZINYL-DICARBONSÄURE ALS SELECTIVE NMDA-REZEPTOR MODULIERENDE MITTEL
ACIDES PHENANTRYL-PIPERAZINYL-DICARBOXYLIQUES UTILES EN TANT QU'AGENTS MODULATEURS DU RECEPTEUR DE NMDA

(30) Priority: 16.12.1999 GB 9929582
(43) Date of publication of application: 02.10.2002
(73) Proprietor: UNIVERSITY OF BRISTOL, Clifton Bristol BS8 1TH (GB); UNIVERSITY OF NEBRASKA BOARD OF REGENTS, Lincoln, NE 68598 (US)
(72) Inventor: MONAGHAN, Daniel, T., Omaha, NE 68104 (US); JANE, David E., Redland, Bristol BS6 6QX (GB); TSE, Heong Wai, Westbury Park, Bristol BS6 7NJ (GB)
(74) Representative: Stevens, Ian Edward
(86) International application number: PCT/US2000/034137
(87) International publication number: WO 2001/044205

(56) References cited:
- EP-A- 0 159 889
- US-A- 5 985 586
- A. L. BULLER, D. T. MONAGHAN: "Pharmacological heterogeneity of NMDA receptors: characterization of NR1a/NR2D heteromers expressed in Xenopus oocytes" EUROPEAN JOURNAL OF PHARMACOLOGY, vol. 320, 1997, pages 87-94, XP000994801 cited in the application

## Description

### Field of the Invention

This invention relates to phenanthryl piperazinyl dicarboxylic acids (PPDAs), processes for synthesizing the same and methods of use thereof *in vivo* and *in vitro.*

### Background of the Invention

NMDA receptors consist of NMDA receptor 1 (NR1) subunits and members of a family of glutamate-binding NR2 subunits (NR2A-D) (Ikeda K, *et al.* (1992) FEBS Lett 313:34 -38; Monyer H, *et al.* (1992) Science 256:1217-1221; Ishii T, *et al.* (1993) J Biol Chem 268:2836 -2843). Recombinant NMDA receptors that contain NR 1 subunits and subunits NR2A or B require a strong depolarization to overcome Mg²⁺ blockade and have high conductances, whereas those with NR2C or D need only modest depolarization to overcome Mg²⁺ blockade and show low conductances (Monyer *et al., id.,* and Monyer H, *et al.* (1994) Neuron 12:529 -540). Native NMDA receptors containing NR2D are estimated to form ~10% of the NMDA receptor population in the cortex of adult rats (Dunah AW, *et al.* (1998) Mol Pharmacol 53:429 -437). Additionally, levels of expression of these subunits are higher in juvenile animals Dunah AW, *et al.* (1996) J Neurochem 67:2335-2345; Wenzel A, *et al.* (1996) J Neurochem 66:1240 -1248), in which long-term depression can most efficiently be produced (Dudek SM, Bear MF (1993) J Neurosci 13:2910 -2918.). CA1 pyramidal cells express mRNA for NR2A, 2B, and 2D in adult humans (Scherzer CR, *et al.* (1998) J Comp Neurol 390:75-90) and in juvenile rats (Kirson ED, *et al.* (1999) J Physiol (Lond) 521:99 -111). Currents attributable to NMDA receptors containing NR2D subunits have also been observed in juvenile CA1 pyramidal cells (Kirson *et al., id.).*

NMDA receptor subpopulations containing these different subunits can be distinguished by competitive NMDA receptor antagonists with different affinities to the glutamate-binding site of the various NR2s (Monaghan DT, *et al.* (1998) Prog Brain Res 116:158 -177).

In the hippocampal CA1 region and the cerebral cortex, both long-term potentiation (LTP) and long-term depression (LTD) can depend on the activation of NMDA receptors, because both can be blocked by the NMDA receptor antagonist D/L-2-amino-5- phosphonovaleric acid (D/L-AP5) (Collingridge GL, *et al.* (1983) J Physiol (Lond) 334:34 -46; Harris EW, *et al.* (1984) Brain Res 323:132-137; Dudek SM, Bear MF (1992) Proc Natl Acad Sci USA 89:4363- 4367; Mulkey RM, Malenka RC (1992) Neuron 9:967-975; Kirkwood A, *et al.* (1993) Science 260:1518 -1521; Christie BR, *et al.* (1996) Learn Mem 3:160 -169; Cummings JA, *et al.* (1996) Neuron 16:825- 833). High-frequency stimulation causes strong activation of the ligand- and voltage-dependent NMDA receptors. A large influx of Ca²⁺ into postsynaptic neurons follows to trigger potentiation. Low-frequency stimulation results in moderate activation of NMDA receptors and a moderate influx of Ca²⁺, leading to depression. An additional mechanism participating in this bi-directional response, however, may be that high- and low-frequency stimulation activate distinct subpopulations of NMDA receptors (Hrabetova S, Sacktor TC (1997) Neurosci Lett 226:107-110).

Various amino acids have become of interest following the discovery that they are able to influence the binding and modulation of certain receptors in the central nervous system (CNS), including the NMDA receptor. Attention has been directed to the identification of novel compounds that selectively bind and activate or block these receptor sites. Such compounds may be used to advantage to treat disorders resulting from CNS malfunction including, for example, various involuntary muscular activity and/or mental and/or affective disorders.

A number of piperazine-2-carboxylic acid and piperazine-2,3-dicarboxylic acid analogues have been synthesised as potential NMDA receptor antagonists. EP-A-0159889 and GB-A-2157685 disclose several such compounds, including 1-(4-bromobenzoyl)-piperazine-2,3-dicarboxylic acid (BrBzPDA). The compounds are, however, relatively weak NMDA receptor antagonists and they are also relatively non-selective for the NMDA receptors, in that they also antagonize alpha-amino-3-hydroxy-5-methylisoxazole-4-propionic acid (AMPA) and kainate-induced depolarizations on neonatal rat motoneurones.

A more potent and selective NMDA receptor antagonist, namely 1-(4-phenylbenzoyl)-piperazine-2,3-dicarboxylic acid, (PBPD), has been described by Buller *et al.,* European Journal of Pharmacology, 320, (1997), 87-94. PBPD displays some NMDA receptor subtype selectivity. Indeed, PBPD displays the opposite selectivity to previously described antagonists such as 4-(3-phosphonoprop-2-enyl)piperazine-2-carboxylic acid (CPP-ene) in that it selectively antagonises NR1/NR2B or NR1/NR2D receptors over those containing NR1/NR2A or NR1/NR2C. However, PBPD is only of moderate potency when compared to previously described antagonists such as CPP-ene.

There remains a need for NMDA receptor antagonists which have high potency and/or exhibit selectivity for particular NMDA receptor subtypes. It is an aim of the invention to provide such compounds

### Summary of the Invention

According to the present invention, there are provided compounds of formula (I) 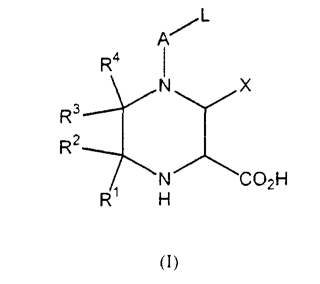 wherein:
L is 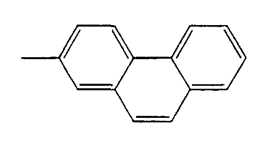 optionally substituted by replacement of one or more of the hydrogen atoms on the phenanthrene ring system by one or more groups which may be the same or different selected from halo, cyano, hydroxy, C₁ to C₆ alkoxy, C₁ to C₆ alkyl, C₂ to C₆ alkenyl, C₂ to C₆ alkynyl, aromatic, carbocyclic and heterocyclic ring systems, wherein the groups from C₁ to C₆ alkyl to heterocyclic may be optionally substituted with one or more groups selected from: halo, cyano, hydroxy, C₁ to C₆ alkoxy, C₁ to C₆ alkyl, C₂ to C₆ alkenyl, C₂ to C₆ alkynyl, aromatic, carbocyclic and heterocyclic ring systems, optionally substituted on the ring by one or more of the same or different groups selected from halo, cyano, hydroxy, nitro, C₁ to C₆ alkyl, C₂ to C₆ alkenyl and C₂ to C₆ alkynyl; and benzyl;
A is CH₂, SO₂ or C=O;
X is CO₂H, PO₃H₂, PO₂H₂, PO₂HR⁵, PO₂HOR⁵, SO₃H, SO₂H, or tetrazole; and
R¹, R² R³, R⁴ and R⁵ are independently selected from H, C₁ to C₆ alkyl, C₂ to C₆ alkenyl, C₂ to C₆ alkynyl, aromatic, carbocyclic and heterocyclic ring systems, optionally substituted on the ring by one or more of the same or different groups selected from halo, cyano, hydroxy, nitro, C₁ to C₆ alkyl, C₂ to C₆ alkenyl and C₂ to C₆ alkynyl and C₁ to C₆ alkyl substituted with aromatic, carbocyclic or heterocyclic ring systems, optionally substituted on the ring by one or more of the same or different groups selected from halo, cyano, hydroxy, nitro, C₁ to C₆ alkyl, C₂ to C₆ alkenyl and C₂ to C₆ alkynyl;
or a pharmaceutically acceptable acid salt or base addition salt or an *in vivo* hydrolysable ester formed by esterification of one or more of the acidic groups in the compound or amide formed by acylation of one or more of the amino groups in the compound thereof.

In a further aspect of the invention, methods of synthesizing compounds of formula (I) are provided.

In yet another aspect of the invention, compounds of formula (I) are employed for the diagnosis and treatment of CNS disorders due to aberrant NMDA receptor activity. Also provided are pharmaceutical compositions comprising compounds of formula (I) for administration to patients in need thereof.

### Brief Description of the Drawing

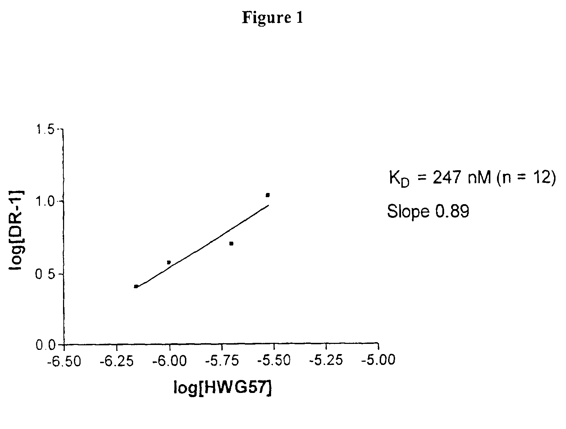

Figure 1 is a graph showing the results of a Schild analysis of NMDA-induced depolarizations in the spinal cord. The data reveal that PPDA, also referred to herein as HWG57, is a competitive antagonist with an affinity (K_{d}) of 247 nM.

Group L in the compounds of formula (I), which is based on a phenanthrene ring system, is optionally substituted with one or more (ie, from one to nine) groups which may be the same or different. Optional substituents on L include, for example, halo, cyano, hydroxy, alkoxy, alkyl, alkenyl, alkynyl, aryl, aralkyl, which latter five groups are all optionally substituted with one or more groups selected from halo, cyano, hydroxy, alkoxy, alkyl, alkenyl, alkynyl, aryl and aralkyl. Preferably, L is unsubstituted.

Preferably, in the compounds of formula (I), A is C=O.

Compounds of formula (I), which are also referred to herein as "compounds of the invention", comprise group X , an acidic moiety. Preferably, X is -CO₂H.

The term "alkyl", as used herein, includes branched and unbranched C₁ to C₆ alkyl (*e.g.*, methyl, ethyl, 1-propyl, 2-propyl, 1-butyl and 2-(2-methylpropyl)) and C₃ to C₆ cycloalkyl *(e.g.,* cyclopropyl, cyclobutyl, cyclopentyl and cyclohexyl). The terms "alkenyl" and "alkynyl" are defined similarly, but the groups to which they refer contain at least one carbon-carbon double bond or triple bond, respectively. "Alkoxy" means alkyl terminated with an oxygen radical (e.g., methoxy, ethoxy, propoxy, butoxy). The term "alkylene" includes C₁ to C₆ alkylene and is a diradical in which the two radical groups are separated by one or more CR'R" groups, wherein R' and R" independently represent H or alkyl.

The term "aryl", as used herein, includes aromatic, carbocylic and heterocyclic ring systems, optionally substituted on the ring by one or more further groups. Aryl thus includes phenyl, naphthyl, pyridyl, thiophenyl and furanyl, for example, all optionally substituted. Suitable substituents on the aryl ring systems include, for example, one or more of the same or different groups selected from halo, cyano, hydroxy, nitro, alkyl, alkenyl and alkynyl. "Aralkyl" means alkyl substituted with aryl *e.g.*, benzyl.

The term "halo", as used herein, covers fluoro, chloro, bromo and iodo. "Haloalkyl" means alkyl substituted with one or more of the same or different halo groups *e.g.*, chloromethyl or trifluoromethyl.

The compounds of the invention may be in the form of pharmaceutically acceptable acid salts or base addition salts. Suitable acid salts include, for example, sodium salts formed by deprotonation of one or more of the acidic groups (*e.g.*, carboxylic acid groups) in the compounds of formula (I). Suitable base addition salts include, for example, salts formed between the compounds of formula (I) and an acid, such as hydrochloric acid for example, by protonation of an amino group. The compound of formula (I) may be in the form of *in vivo* hydrolysable esters *(e.g.,* formed by esterification of one or more of the acidic groups in the compounds of the invention) or amides (*e.g.*, formed by acylation of one or more of the amino groups in the compounds of the invention).

Compounds of formula (I) have one or more chiral centers and may be in the form of a racemic mixture of enantiomers, a mixture of enantiomers substantially enriched in one enantiomer or a substantially pure enantiomer. Similarly, compounds of formula (I) may be in the form of a single diastereomer or mixtures of diastereomers. It is preferred for compounds of formula (I) that groups X and -CO₂H, as shown in formula (I) are *cis* to each other on the six-membered piperazine ring.

Preferred compounds of the invention include 1-(phenanthrene-2-ylcarbonyl)piperazine-2,3-dicarboxylic acid, and, more preferably, *cis*-1-(phenanthrene-2-ylcarbonyl)piperazine-2,3-dicarboxylic acid.

Compounds of formula (I) may exist in one or more tautomeric forms, all of which are included in the present invention.

The compounds of the invention may be produced by a number of routes. In another aspect, the present invention provides a process for preparing the compounds which comprises the reaction of a compound of formula (II) 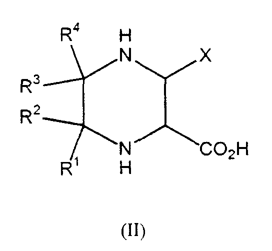 with a compound of formula L-A-M, wherein: R¹, R², R³, R⁴, X, L and A are as defined above for compounds of formula (I) and M is a leaving group.

The process of the invention is typically carried out in a solvent for a time and at a temperature to produce sufficient amounts of the compound of formula (I). Preferably, the process includes the steps of separating the compound of formula (I) from the reaction mixture and then purifying the compound of formula (I). The process of the invention may involve, for example, the use of a compound of formula L-A-M in which M is halo, when A is CH₂, SO₂ or CO, or in which M is -OH, -OR"' or -OC(O)OR"' (wherein R"' is alkyl, aryl or aralkyl), when A is CO. In the case in which A is CO, the process may comprise the reaction of a compound of formula (II) with an acyl halide of formula L-COC1, in a polar solvent such as 1,4-dioxane, at room temperature for 1 to 4 hours. The process may comprise the separation of the compound of formula (I) from the reaction mixture by ion exchange resin chromatography and, optionally, further purification by recrystallisation from a suitable solvent, *e.g.*, water.

Compounds of formula **(II)** are commercially available or are obtainable from commercially available compounds by standard routes. For example, compounds of formula (II) in which X is CO₂H may be prepared from the corresponding pyrazine compound by reduction with hydrogen in the presence of a catalyst (*e.g.* PtO₂).

In one preferred embodiment, (±)-cis-1-(10-bromophenanthrene-2-carbonyl)piperazine-2,3-dicarboxylic acid (bromoPPDA, **5)** was synthesised according to Scheme 1. Phenanthrene-2-carboxylic acid **(1)** was brominated with bromine in acetic acid at 70ºC to give the bromo derivative **(2)** which was converted into the acid chloride **(3)** using thionyl chloride. The acid chloride **(2)** was coupled to (_)-cis-piperazine-2,3-dicarboxylic acid **(4)** in an aqueous sodium hydroxide / dioxan mixture to give the target molecule **(5)** which was purified by crystallization from water.

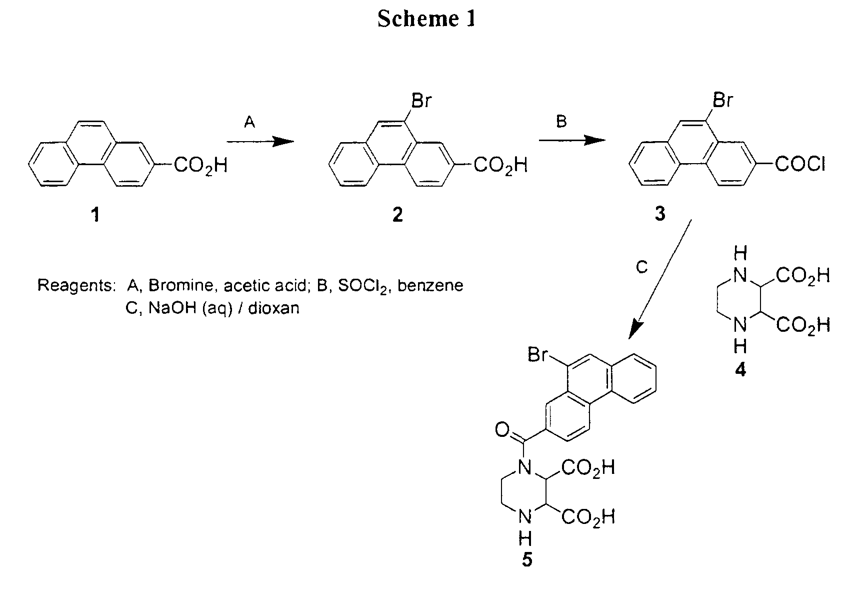

Compounds of formula (I) possess NMDA receptor modulating activity *in vitro* and *in vivo.* As such, they may be used to advantage in the treatment of CNS disorders arising from aberrant NMDA receptor activity.

The present invention also provides pharmaceutical compositions comprising a compound of the invention together with a pharmaceutically acceptable diluent or carrier. The pharmaceutically acceptable diluents or carriers which are suitable for use in any given composition depends upon the intended mode of administration of the composition and will be well-known to those skilled in the art.

The compounds of the invention may be administered parenterally or orally, *e.g.*, intravenously for acute treatment, or subcutaneously, or orally for chronic treatment. Compounds of the invention may be formulated for clinical use in suitable vehicles, normally as preparations of a water-soluble salt, though preparations of low water solubility, and optionally in association with physiologically tolerable emulsifying agents.

For enhanced efficacy, it may be necessary for compounds of the invention to penetrate the blood brain barrier. In such cases, the compounds of the present invention may be administered in excess amounts to ensure that appropriate concentrations of the compounds are achieved within the brain for the therapeutic effect desired. Accordingly, this will influence the concentration of the active compounds in the compositions of the present invention. Considerations of this type indicate that compositions might contain the active compound in a concentration such that a conventional dosage volume would provide the subject with up to about 150-350 mg/kg body weight and more preferably 200 mg/kg body weight. When the compounds are to be administered by the intravenous or subcutaneous route, dosages in the region of about 1-20 mg/kg body weight are suitable for the more active compounds and/or for those substances with a high lipophilic to hydrophilic balance.

Also provided by the present invention is a method of treating a patient suffering from a disorder of the CNS arising from aberrant NMDA receptor behavior which comprises administering to the patient a therapeutically effective amount of a compound of the invention.

Further provided by the present invention is the use of a compound of the invention in the manufacture of a medicament for the treatment of a disorder of the CNS arising from aberrant NMDA receptor behavior.

Disorders of the CNS which may be treated according to the invention include epilepsy, pain-related disorders, narcotic-related disorders (*e.g.* narcotic tolerance) and disorders arising from neuronal cell death following ischaemia or stroke or head and/or spinal cord injury or HIV infection. Further disorders which may be treated according to the present invention include psychiatric disorders (such as schizophrenia) and neurodegenerative diseases such as Alzheimer's disease, Parkinson's disease and Huntington's disease.

The compounds of the invention are antagonists at NMDA receptor sites in the CNS and show greater potency when compared to compounds of the prior art. Furthermore, the claimed compounds also show greater selectivity for NMDA receptor subtypes, such as, for example, for NR2C or NR2D over NR2A or NR2B. The selectivity of the compounds provides therapeutic advantages over known NMDA antagonists since enhanced selectivity reduces unwanted side-effects. Adverse side-effects encountered with prior art compounds include memory loss, psychotomimetic effects and loss of motor co-ordination. The alleviation of any of these side-effects is clearly advantageous.

The compounds of the invention may also be used as research tools for the study of NMDA receptors. When used as research tools, the compounds of the invention may be detectably labelled, e.g., by the incorporation of one or more ³H atoms (replacing ¹H atoms) into the compounds of the invention. Preferably, the radiolabelling is by substitution of one or more ¹H atoms (*e.g.*, by ³H) on the phenanthrene ring. Alternatively, such detectable labels may include fluorophore conjugates, chemiluminescent conjugates, and the like.

The invention will now be illustrated by reference to the following nonlimiting examples.

### EXAMPLE 1

### Preparation of Piperazine-2,3-dicarboxylic acid

Pyrazine-2,3-dicarboxylic acid (21.27 g, 0.127 mol) was dissolved in an aqueous solution of sodium hydroxide (10.12 g, 0.253 mol) to give disodium pyrazine-2,3-dicarboxylic acid. The aqueous solution of the acid was hydrogenated under 40 p.s.i of hydrogen in the presence of platinum (IV) oxide catalyst (0.5 g) for a period of 3 days. The reaction mixture was filtered and the solvent removed to give an oil. The oil was bound onto an ion exchange resin (Dowex-AG-50 H⁺ form) column, and was eluted with water until the eluant had a pH of 5. The column was eluted with a 10 % aqueous solution of pyridine, the ninhydrin active fractions were combined and the solvent removed *in vacuo.* The solid was purified by crystallisation to give the title compound (25.64 g, 95.6 %).

### EXAMPLE 2

### Preparation of 2-Carboxyphenanthrene

Bromine (4.7 ml, 0.091 mol) was added to an ice-cold solution of sodium hydroxide (12.7 g, 0.318 mol) in water (70 ml) to form a basic solution of sodium hypobromite. The sodium hypobromite solution was added to a stirred solution of 2-acetylphenanthrene in dioxane (70 ml) at 60-65 °C for 15 min. Excess sodium hypobromite was removed by the addition of 10 % solution of sodium metabisulphite (20 ml). Water (300 ml) was added and approximately 100 ml of solvent was removed under reduced pressure. The reaction mixture was acidified with concentrated HCl. The precipitate formed was filtered and washed with water to give the title compound as a solid (4.01 g, 99.2 %).

### EXAMPLE 3

### Preparation of (±)-cis-1-(Phenanthrene-2-ylcarbonyl)piperazine-2,3-dicarboxylic acid (PPDA)

2-Carboxyphenanthrene (2 g, 0.009 mol) was suspended in dry benzene (50 ml) and heated under reflux in the presence of thionyl chloride (5 ml) for 5 hr. The solvent was removed and the corresponding acid chloride was used without further purification. Piperazine-2,3-dicarboxylic acid (0.508 g, 0.003 mol) was dissolved in a 0.991 M aqueous sodium hydroxide solution (9.4 ml, 0.009 mol), dioxane (10 ml) was added and the solution was cooled to 0° C. A solution of the acid chloride (0.84 g, 0.0035 mol) in dioxane (10 ml) was added to the reaction mixture. The reaction was warmed to room temperature and stirred for 2-3hr. The reaction mixture was acidified to pH 2-3 with aqueous HCl. The solid was filtered, dissolved in dioxane/H₂O (50:50) and bound to an ion exchange resin (Dowex-AG50 H⁺ form) chromatography column. It was eluted with dioxane/ H₂O (50:50) until organic impurities are removed. The column was eluted with 10 % aqueous pyridine and the ninhydrin fractions were combined. The solvent was removed and the solid was crystallised from H₂O to give a white solid (0.128 g, 12 %). m.p. = 218.1-221.7 °C (dec.); ¹H NMR (300MHz, NaOD/ D₂O) 7.7-8.8 (m, 9H, Ar), 5.6 (d, 0.5H, CH-CO,H, J_{AB} = 2.6Hz), 4.8 (d, 0.5H, CH-CO₂H, J_{AB} = 2.6Hz), 4.4 (d, 0.4H, CH-CO₂H, J_{AB} = 12.0Hz), 3.6 (d, 0.4H, CH-CO₂H, J_{AB} = 12.0Hz), 2.5-3.8 (m, 4H, N-CH₂CH₂-NH); Elemental analysis calculated for C₂₁H₁₈N₂O₅. 2.3H₂O C: 60.07, H: 5.44, N: 6.67. Found C: 60.06, H:5.38, N: 6.85. The *cis-* isomerism was confirmed by COSEY ¹H NMR.

### EXAMPLE 4

### Preparation of 10-bromophenanthrene-2-carboxylic acid (2)

Phenanthrene-2-carboxylic acid (1, 0.116 g, 0.5 mmol) was dissolved in acetic acid at 70ºC and then bromine (0.027 ml, 0.5 mmol) was added. The mixture was stirred at 70°C overnight. The following day, bromine (0.027 ml, 0.5 mmol) was added and the mixture was refluxed for 6 h and then allowed to cool. The resulting precipitate was collected by filtration. ¹H NMR (270 MHz, DMSO): _ 7.8 (m, 2H), 8.1 (d, 1H), 8.25 (d, 1H), 8.45 (s, 1H), 8.9 (s, 1H), 8.95 (m, 1H) and 9.05 (d, 1H).

### EXAMPLE 5

### Preparation of (±)-cis-1-(10-bromophenanthrene-2-carbonyl)piperazine-2,3-dicarboxylic acid (5)

10-Bromophenanthrene-2-carboxylic acid (**2**, 0.663 g, 2.2 mmol) was added to benzene (50 ml) containing excess thionyl chloride and the mixture heated under reflux overnight. The following day, the mixture was evaporated under reduced pressure and the resulting acid chloride **(3)** used without further purification. (_)-Cis-piperazine-2,3-dicarboxylic acid (**4**, 0.46 g, 2.6 mmol) was added to 0.991 M aqueous sodium hydroxide (8 ml, 8 mmol) and dioxan (10 ml) and the mixture was cooled to 0ºC. A solution of the crude acid chloride **(3)** in dry dioxan was then added and the mixture was stirred at 0ºC for 5 min and then at room temperature overnight. The following day, the reaction mixture was reduced to ~ half the volume by evaporation under reduced pressure and was then acidified with 6 M aqueous hydrochloric acid. The resulting precipitate was collected by filtration, heated in dioxan (50-100 ml) and filtered. This procedure was then repeated twice. The resulting solid was added to water just below the boiling point and stirred in order to dissolve water soluble byproducts. The insoluble material was collected by filtration, washed with dioxan and dried to give (±)-cis-1-(10-bromophenanthrene-2-carbonyl)piperazine-2,3-dicarboxylic acid **(5)** as a white solid. ¹H NMR (270 MHz, D₂O/NaOD): _ 2.5-3.4 (m, 4H), 4.3 (d, 0.5 H), 5.5 (d, 0.5 H), 7.2-7.6 (m), 7.65-7.8 (m), 7.75 (s) 7.95-8.2 (m), 8.1 (s) and 8.25-8.5 (m).

### EXAMPLE 6

### PPDAs Differentiate between Ionotropic Receptors in Motoneurones of the Neonatal Rat Spinal Cord

Hemisected neonatal rat spinal cords (Evans and Watkins, European Journal of Pharmacology, 50: 123-129, 1978) were isolated and wire electrodes were placed under the exposed ventral roots. The preparation was superfused with standard Ringers (containing in mM: NaCl 118, NaHCO₃ 25, KCl 3, CaCl₂ 2.5, D-glucose 12 and gassed with 95% O₂/5%CO₂). To record agonist-induced responses, 0.1 *µ*M tetrodotoxin was included to block synaptic transmission and associated depolarizations. Agonist (N-methyl-D-aspartate (NMDA)) was superfused for 60 - 120 seconds at a rate of 1ml/minute. The relative positive potentials recorded by the distal (to spinal cord) wire electrode compared to the proximal electrode was taken as an indication of motor neuron cell body depolarization. Antagonism of agonist-induced responses was observed by co-application of the antagonist PPDA.

Figure 1 shows the effect of PPDA on ionotropic receptors on motoneurones in the neonatal rat spinal cord. This figure is a graph of a Schild analysis of NMDA-induced depolarizations in the spinal cord showing that PPDA is a competitive antagonist with an affinity (Kd) of 247 nM. The DR is the dose ratio of agonist concentration in presence of antagonist that produces a comparable response when compared to treatment with the agonist alone. Thus, higher agonist concentrations are necessary (higher DR) to reverse the block of higher concentrations of PPDA indicating that the blockade is one of a competitive nature. PPDA is as potent as CPP-ene as an antagonist of NMDA-induced depolarizations in the spinal cord.

AMPA and kainate are protypical agonists which show selectivity for, and predominantly activate, the AMPA and kainate subtypes of glutamate receptors. In additional assays, PPDA was tested at a concentration of 5 µM (which is approximately 20 times the K_{d} value for the antagonism of NMDA receptors) as an antagonist of AMPA (6 µM)-, kainate (50 µM)- and NMDA (50 µM)-induced depolarizations (approximate EC₅₀ concentrations used) on neonatal rat motoneurones. The results are shown below. Results:
88% antagonism of NMDA
15% antagonism of AMPA
21% antagonism of kainate

These results show that PPDA selectively binds the NMDA subtype of glutamate receptor.

PPDA (50 µM) also had no effect on depolarizations following administration of (1S,3R)-1-amino-1,3-cyclopentane dicarboxylic acid (ACPD; 30 µM) . APCD is a Group I metabotropic glutamate (mGlu) receptor agonist. The recited values were determined by using quantitative receptor autoradiography of [³H]-glutamate labeled NMDA receptors in rat brain tissue sections. The data shows that PPDA is selective NMDA receptor antagonist having weaker antagonist activity on AMPA/kainate receptors and substantially no antagonist activity on group I mGlu receptors.

The potency of PPDA for native NMDA receptors was also investigated in a *Xenopus* oocyte system. The results are shown in Table 1.

### PPDAs Differentiate between Subclasses of NMDA Receptors in vitro in Xenopus oocytes.

**Table 1**

| Native NMDA Receptor Potency | PPDA Kᵢ (µM) |
|---|---|
| Medial Striatum (NR2B) | 0.54 +/- 0.04 |
| Cerebellum (NR2A/NR2C) | 7.2 and 0.39 µM, respectively |
| Midline thalamus (NR2B/NR2D) | 0.31 +/- 0.03 |

### EXAMPLE 7

### PPDAs Differentiate between Ionotropic Receptors in vitro in Xenopus oocytes

RNA translation and transcription in *Xenopus* oocytes and electrophysiological recordings were performed as previously described (Monaghan DT, Larson H (1997) J Pharmacol Exp Ther 280:614 -620). Plasmids were linearized with *No*tI (NR1a), *Eco*RI (NR2A, NR2C, and NR2D), or *Sa*ll (NR2B) and transcribed *in vitro* using the mMessage mMachine RNA polymerase transcription kit (Ambion, Austin, TX). NR1a was mixed 1:3 with NR2A, NR2B, NR2C, or NR2D RNA, and 2-50 ng of this mixture was injected into oocytes. Agonist-evoked responses were measured using a standard two-microelectrode voltage clamp (model OC-725B Oocyte Clamp; Warner Instruments, Hamden, CT) at a holding potential of -60 mV. Glutamate (10 µM) and glycine (10 µM) were applied until stable plateau responses were obtained; (±)-cis-1-(phenanthren-2-yl-carbonyl)-piperazine-2,3-dicarboxylic acid (PPDA) (0.1, 0.3, 1, 3, 10, or 30 µM) was then applied until steady-state blockade was obtained, followed by antagonist washout and full agonist responses. Current responses were captured and analyzed with AxoData (Axon Instruments, Foster City, CA) and GraphPad Prism (ISI Software, San Diego, CA) software. Kᵢ values were corrected for agonist affinity according to the Cheng-Prusoff equation. Data in Table 3 are expressed as mean Kᵢ ± SEM. PPDA was found to have little effect on native non-NMDA glutamate receptors. Autoradiography, performed as described by Monaghan DT in: Receptor autoradiography: principles and practice (Wharton J, Polak JM, eds), pp 171-193. New York: Oxford UP. (1993), showed that at 10 µM, PPDA inhibited 44.6 ± 1.3% of 100 nM [³H] 6-cyano-7-nitroquinoxaline-2,3-dione (CNQX) binding to native AMPA receptors and 9.3 ± 6.7% of 25 nM [³H]kainate (*n* = 3).

The selectivity of PPDA for different NMDA receptor subtypes was investigated. The results are shown in Table 2.

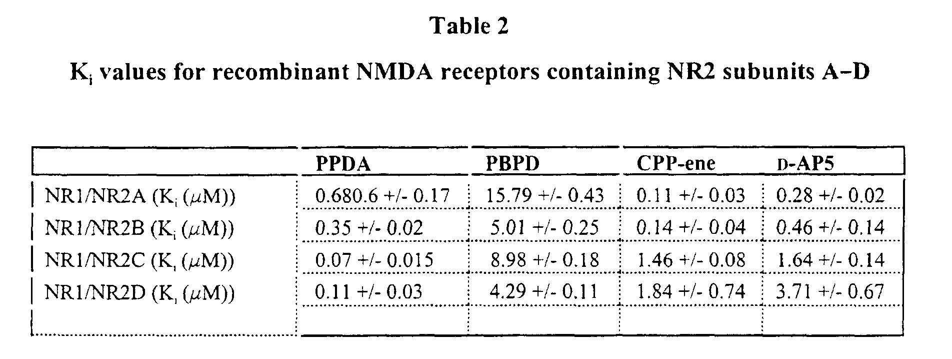

In Table 2, the Kᵢ values were obtained from NR2 subunits recombinantly expressed with NR1a subunits to form receptors in *Xenopus* oocytes. The Kᵢ values for D-AP5 and D-CPPene are from Buller AL, Monaghan DT (1997) Eur J Pharmacol 320:87-94 (mean ± SEM; *n* = 4-6); in the Kᵢ values for PPDA , *n* = 3. Differences among the agents were expressed by normalization to PPDA [(2C + 2D) Kᵢ /(2A + 2B) Kᵢ ^{nL PPDA}] and obtained by dividing the ratios for each drug by the ratio for PPDA.

## Claims

1. Compound of formula (I) 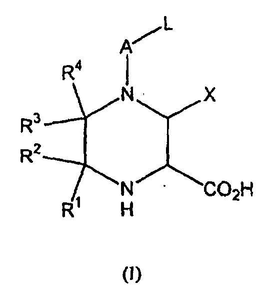 wherein:
L is 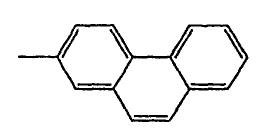 optionally substituted by replacement of one or more of the hydrogen atoms on the phenanthrene ring system by one or more groups which may be the same or different selected from halo, cyano, hydroxy, C₁ to C₆ alkoxy, C₁ to C₆ alkyl, C₂ to C₆ alkenyl, C₂ to C₆ alkynyl, aromatic, carbocyclic and heterocyclic ring systems, wherein the groups from C₁ to C₆ alkyl to heterocyclic may be optionally substituted with one or more groups selected from: halo, cyano, hydroxy, C₁ to C₆ alkoxy, C₁ to C₆ alkyl, C₂ to C₆ alkenyl, C₂ to C₆ alkynyl, aromatic, carbocyclic and heterocyclic ring systems, optionally substituted on the ring by one or more of the same or different groups selected from halo, cyano, hydroxy, nitro, C, to C₆ alkyl, C₂ to C₆ alkenyl and C₂ to C₆ alkynyl; and benzyl;
A is CH₂, SO₂ or C=O;
X is CO₂H, PO₃H₂, PO₂H₂, PO₂HR⁵, PO₂HOR⁵, SO₃H, SO₂H, or tetrazole; and
R¹, R², R³, R⁴ and R⁵ are independently selected from H, C₁ to C₆ alkyl, C₂-C₆ alkenyl, C₂-C₆ alkynyl, aromatic, carbocyclic and heterocyclic ring systems, optionally substituted on the ring by one or more of the same or different groups selected from halo, cyano, hydroxy, nitro, C₁ to C₆ alkyl, C₂ to C₆ alkenyl and C₂ to C₆ alkynyl and C₁ to C₆ alkyl substituted with aromatic, carbocyclic or heterocyclic ring systems, optionally substituted on the ring by one or more of the same or different groups selected from halo, cyano, hydroxy, nitro, C₁ to C₆ alkyl, C₂ to C₆ alkenyl and C₂ to C₆ alkynyl;
or a pharmaceutically acceptable acid salt or base addition salt or an in vivo hydrolysable ester formed by esterification of one or more of the acidic groups in the compound or amide formed by acylation of one or more of the amino groups in the compound thereof.

2. Compound of Claim 1, wherein A is C=O.

3. Compound as claimed in Claim 1 or Claim 2, wherein X is CO₂H.

4. Compound as claimed in any one of Claims 1 to 3, wherein R¹, R², R³, R⁴ and, if present, R⁵ are all H.

5. Compound as claimed in any one of Claims 1 to 4, wherein L is unsubstituted.

6. Compound as claimed in Claim 1 wherein the groups X and -CO₂H are *cis* to each other.

7. Compound as claimed in Claim 1 which is *cis-*1-(phenanthrene-2-carbonyl)piperazine-2,3-dicarboxylic acid.

8. Compound as claimed in any one of Claims 1 to 7 which is radiolabelled.

9. Compound of any one of Claims 1 to 8 for use in medicine.

10. Pharmaceutical composition comprising a compound of any one of Claims 1 to 9 together with a pharmaceutically acceptable diluent or carrier.

11. Process for producing a compound of any one of Claims 1 to 8 which comprises the reaction of a compound of formula (II): 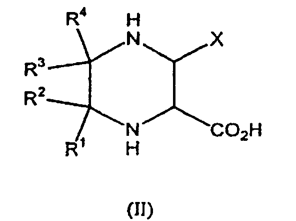 with a compound of formula L-A-M wherein: R¹, R², R³, R⁴, X, L and A are as defined in Claim 1; and M is a leaving group.

12. A process as claimed in Claim 11, wherein M is halo.

13. Use of a compound of any one of Claims 1 to 7 in the manufacture of a medicament far the treatment of a disorder of the CNS.

14. Use as claimed in Claim 13, wherein the disorder of the CNS is selected from the group consisting of epilepsy, pain-related disorders, narcotics-related disorders, disorders arising from neuronal cell death following ischaemia or stroke or head and/or spinal cord injury or HIV infection, psychiatric disorders, schizophrenia, neurodegenerative disorders, Alzheimer's disease, Parkinson's disease and Huntington's disease.

15. The compound according to claim 1, (±)-cis-1-(10-bromophenanthrene-2-carbonyl)piperazine-2,3-dicarboxylic acid.

## Patentansprüche

1. Verbindung der Formel (1) 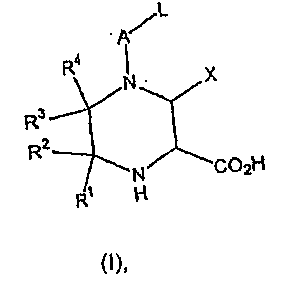 wobei:
L 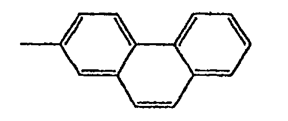 ist, das optional durch Austausch eines oder mehrerer der Wasserstoffatome des Phenanthrenringsystems durch eine oder mehrere Gruppen substituiert ist, die dieselben oder unterschiedliche Gruppen sein können, welche ausgewählt sind aus Halogen-, Cyano-, Hydroxy-, C₁ bis C₆ Alkoxy-,_{.} C₁ bis C₆ Alkyl-, C₂ bis C₆ Alkenyl-, C₂ bis C₆ Alkynyl-, aromatischen, carbozyklischen und heterozyklischen Ringsystemgruppen, wobei die Gruppen von der C₁ bis C₆ Alkylgruppe bis zu der heterozyklischen optional durch eine oder mehrere Gruppen substituiert sein können, die ausgewählt sind aus Halogen-, Cyano-, Hydroxy-, C₁ bis C₆ Alkkoxy-, C₁ bis C₆ Alkyl-, C₂ bis C₆ Alkenyl-, C₂ bis C₆ Alkynyl-, aromatischen, carbozyklischen und heterozyklischen Ringsystemgruppen, die an dem Ring optional durch eine oder mehrere derselben oder unterschiedlicher Gruppen substituiert sind, die ausgewählt sind aus Halogen-, Cyano-, Hydroxy-, Nitro-, C₁ bis C₆ Alkyl-, C₂ bis C₆ Alkenyl- und C₂ bis C₆ Alkynylgruppen; und Benzylgruppen,
A CH₂, SO₂ oder C=O ist;
X CO₂H, PO₃H₂, PO₂H₂, PO₂HR⁵ PO₂HOR⁵, SO₃H, SO₂H oder Tetrazol ist; und
R¹, R², R³, R⁴ und R⁵ unabhängig voneinander ausgewählt sind aus H, C₁ bis C₆ Alkyl, C₂ bis C₆ Alkenyl, C₂ bis C₆ Alkynyl, aromatischen, carbozyklischen und heterozyklischen Ringsystemen, die an dem Ring optional durch eine oder mehrere derselben oder unterschiedlicher Gruppen substituiert sind, welche ausgewählt sind aus Halogen-, Cyano-, Hydroxy-, Nitro-, C₁ bis C₆ Alkyl-, C₂ bis C₆ Alkenyl- und C₂ bis C₆ Alkynylgruppen, und C₁ bis C₆ Alkyl substituiert mit aromatischen, carbozyklischen oder heterozyklischen Ringsystemen, die an dem Ring optional durch eine oder mehrere derselben oder unterschiedlicher Gruppen substituiert sind, welche ausgewählt sind aus Halogen-, Cyano-, Hydroxy-, Nitro-, C₁ bis C₆ Alkyl-, C₂ bis C₆ Alkenyl- und C₂ bis C₆ Alkynylgruppen,
oder ein pharmazeutisch akzeptables saures Salz oder Baseadditionssalz oder ein *in* vivo hydrolysierbarer Ester, der durch Veresterung von einer oder mehrerer der Säuregruppen der Verbindung gebildet wird, oder ein Amid derselben, das durch Azylierung einer oder mehrerer der Aminogruppen der Verbindung gebildet wird.

2. Verbindung nach Anspruch 1, wobei A C=O ist.

3. Verbindung nach Anspruch 1 oder Anspruch 2, wobei X CO₂H ist.

4. Verbindung nach einem der Ansprüche 1 bis 3, wobei R¹, R², R³, R⁴ und falls vorhanden R⁵ alle H sind.

5. Verbindung nach einem der Ansprüche 1 bis 6, wobei L unsubstituiert ist.

6. Verbindung nach Anspruch 1, wobei die Gruppen X und -CO₂H *cis* zueinander sind.

7. Verbindung nach Anspruch 1, die *cis*-1-(Phenanthren-2-carbonyl)piperazin-2,3-dicarboxylsäure ist.

8. Verbindung nach einem der Ansprüche 1 bis 7, die radioaktiv markiert ist.

9. Verbindung nach einem der Ansprüche 1 bis 8 zur Verwendung in der Medizin.

10. Pharmazeutische Zusammensetzung, die eine Verbindung nach einem der Ansprüche 1 bis 9 zusammen mit einem pharmazeutisch akzeptablen Verdünnungsmittel oder Träger aufweist.

11. Verfahren zur Herstellung einer Verbindung nach einem der Ansprüche 1 bis 8, dass die Reaktion einer Verbindung der Formel II: 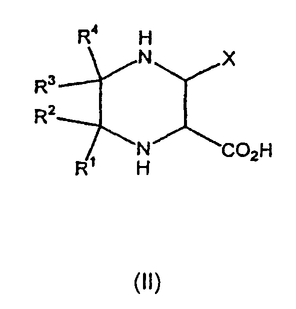 mit einer Verbindung der Formel L-A-M aufweist, wobei: R¹, R², R³, R⁴, X, L und A dieselben wie in Anspruch 1 definiert sind und M eine sich ablösende Gruppe ist.

12. Verfahren nach Anspruch 11, wobei M eine Halogengruppe ist.

13. Verwendung einer Verbindung nach einem der Ansprüche 1 bis 7 bei der Herstellung eines Medikaments für eine Behandlung von Störungen des zentralen Nervensystems.

14. Verwendung nach Anspruch 13, wobei die Störung des zentralen Nervensystems ausgewählt ist aus der Gruppe, die besteht aus Epilepsie, schmerzbezogenen Störungen, Narkotika-bezogenen Störungen, Störungen, die aufgrund von Nervenzellentod nach Ischämieerkrankungen oder Schlaganfällen oder Kopf- oder Rückenmarksverletzungen oder HIV-Infektionen auftreten, psychiatrischen Störungen, Schizophrenie, neurodegenerative Störungen, Alzheimerkrankheit, Parkinsonkrankheit und Huntingtonkrankheit.

15. Verbindung nach Anspruch 1, die (±)-cis-1-(10-Bromphenanthren-2-carbonyl)piperazin-2,3-dicarboxylsäure ist.

## Revendications

1. Composé répondant à la formule (I) : 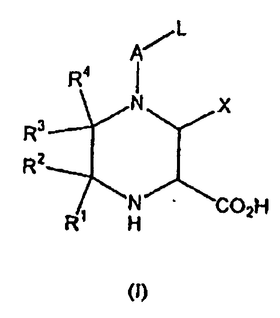 dans laquelle
L est 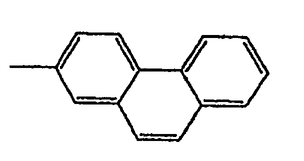 éventuellement substitué en remplaçant un ou plusieurs atomes d'hydrogène sur le système du cycle phénanthrène par un ou plusieurs groupes, qui peuvent être identiques ou différents, choisis parmi un atome d'halogène, un groupe cyano, un groupe hydroxy, un groupe alcoxy en C₁ à C₆, un groupe alkyle en C₁ à C₆, un groupe alcényle en C₂ à C₆, un groupe alcynyle en C₂ à C₆, des systèmes de cycles aromatiques, carbocycliques et hétérocycliques, dans lesquels les groupes du alkyle en C₁ à C₆ aux cycles hétérocycliques peuvent éventuellement être substitués par un ou plusieurs groupes choisis parmi : un atome d'halogène, un groupe cyano, un groupe hydroxy, un groupe alcoxy en C₁ à C₆, un groupe alkyle en C₁ à C₆, un groupe alcényle en C₂ à C₆, un groupe alcynyle en C₂ à C₆, des systèmes de cycles aromatiques, carbocycliques et hétérocycliques, éventuellement substitués sur le cycle par un ou plusieurs groupes, identiques ou différents, choisis parmi un atome d'halogène, un groupe cyano, un groupe hydroxy, un groupe nitro, un groupe alkyle en C₁ à C₆, un groupe alcényle en C₂ à C₆ et un groupe alcynyle en C₂ à C₆; et un groupe benzyle ;
A est CH₂, SO₂ ou C=O;
X est CO₂H, PO₃H₂, PO₂H₂, PO₂HR⁵, PO₂HOR⁵, SO₃H, SO₂H ou un groupe tétrazole; et
R¹, R², R³, R⁴ et R⁵ sont indépendamment choisis parmi H, un groupe alkyle en C₁ à C₆, un groupe alcényle en C₂ à C₆, un groupe alcynyle en C₂ à C₆, des systèmes de cycles aromatiques, carbocycliques et hétérocycliques, éventuellement substitués sur le cycle par un ou plusieurs groupes, identiques ou différents, choisis parmi un atome d'halogène, un groupe cyano, un groupe hydroxy, un groupe nitro, un groupe alkyle en C₁ à C₆, un groupe alcényle en C₂ à C₆ et un groupe alcynyle en C₂ à C₆, un groupe alkyle en C₁ à C₆ éventuellement substitué par des systèmes de cycles aromatiques, carbocycliques et hétérocycliques, éventuellement substitués sur le cycle par un ou plusieurs groupes, identiques ou différents, choisis parmi un atome d'halogène, un groupe cyano, un groupe hydroxy, un groupe nitro, un groupe alkyle en C₁ à C₆, un groupe alcényle en C₂ à C₆ et un groupe alcynyle en C₂ à C₆ ;
ou un sel acide, un sel d'addition basique, un ester hydrolysable in vivo formé par l'estérification de un ou plusieurs groupes acides du composé ou un amide formé par acétylation de un ou plusieurs groupes amino du composé, pharmaceutiquement acceptable de celui-ci.

2. Composé selon la revendication 1, dans lequel A est C=O.

3. Composé selon la revendication 1 ou 2, dans lequel X est CO₂H.

4. Composé selon l'une quelconque des revendications 1 à 3, dans lequel R¹, R², R³, R⁴ et R⁵, si présent, sont tous H.

5. Composé selon l'une quelconque des revendications 1 à 6, dans lequel L n'est pas substitué.

6. Composé selon la revendication 1, dans lequel les groupes X et -CO₂H sont en position cis l'un par rapport à l'autre.

7. Composé selon la revendication 1, qui est l'acide cis-1-(phénanthrène-2-carbonyl)pipérazine-2,3-dicarboxylique.

8. Composé selon l'une quelconque des revendications 1 à 7, qui est radiomarqué.

9. Composé selon l'une quelconque des revendications 1 à 8, pour une utilisation dans le domaine médical.

10. Composition pharmaceutique comprenant un composé selon l'une quelconque des revendications 1 à 9, ainsi qu'un diluant ou un support pharmaceutiquement acceptable.

11. Procédé de production d'un composé selon l'une quelconque des revendications 1 à 8, comprenant la réaction d'un composé répondant à la formule (II) : 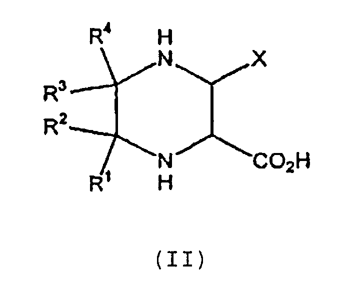 avec un composé répondant à la formule L-A-M dans lesquelles R¹, R², R³, R⁴, X, L et A sont tels que définis dans la revendication 1 ; et M est un groupe partant.

12. Procédé selon la revendication 11, dans lequel M est un atome d'halogène.

13. Utilisation d'un composé selon l'une quelconque des revendications 1 à 7, pour la préparation d'un médicament destiné au traitement d'un trouble du système nerveux central.

14. Utilisation selon la revendication 13, dans laquelle le trouble du système nerveux central est choisi dans le groupe constitué de l'épilepsie, de troubles liés à une douleur, de troubles liés à l'utilisation de narcotiques, des troubles dus à une mort cellulaire neuronale suite à une ischémie, une attaque, un traumatisme crânien et/ou une lésion de la moelle épinière ou une infection à VIH, de troubles psychiatriques, d'une schizophrénie, de troubles neurodégénératifs, de la maladie d'Alzheimer, de la maladie de Parkinson et de la maladie d'Huntington.

15. Composé selon la revendication 1, qui est l'acide (±)-cis-(10-bromophénanthrène-2-carbonyl)-pipérazine-2,3-dicarboxylique.
